# EUROPEAN PATENT SPECIFICATION

(11) **EP 1 260 198 B1**
(45) Date of publication and mention of the grant of the patent: **25.10.2006**
(21) Application number: 02010960.9
(22) Date of filing: 16.05.2002
(51) Int. Cl.: A61F 2/32, A61F 2/30, A61L 27/30

(54) **Movable joint**
Gelenk
Articulation

(30) Priority: 17.05.2001 US 859352
(43) Date of publication of application: 27.11.2002
(73) Proprietor: Noble Medical Coatings, L.L.C., Cleveland, Ohio 44110 (US)
(72) Inventor: Corl, Harry E., III., Twinsburg, OH 44087 (US); Noble, Lawrence E., Scottsdale, AZ 85258 (US)
(74) Representative: Kirschner, Klaus Dieter

(56) References cited:
- WO-A-01/05337
- US-A- 1 864 013
- US-A- 4 335 924
- US-A- 5 571 191
- US-A- 5 660 482
- US-A- 5 824 098

## Description

The present invention relates generally to the field of movable joints. In particular, the present invention involves an improved movable joint having a chromium outer surface and a method for coating the surface of a movable joint.

Movable joints have been utilized in many different technical areas, from medical implants to automobile parts, with each technical area having different, important characteristics. In some applications, the amount of-constant load that a joint can maintain over a long duration is important. In other applications, the amount of extreme load that a joint can maintain over a short period of time may be important. In still other applications, the wear resistance of the joint when the parts of the joint are in relatively constant movement is important. Most applications require a mix of these important factors.

One such application is the use of a ball type joint to replace a natural joint in a human or animal. Ball joints have proven useful in this application because, like the natural joint that the implant is replacing, the joint provides a wide range of motion. However, under these conditions, it is important to have a joint that can be in relatively constant motion and exposed to differing loads without becoming worn, thereby, requiring the joint to be replaced. Since the replacement of the joint is accomplished through invasive surgery, the longer the joint can be utilized without repair or replacement the less risk of injury from the invasive surgery or from complications therefrom.

The present invention may be utilized with any movable joint, but is particularly applicable to ball-type joints. A movable ball joint is typically comprised of two main parts; a ball portion and a socket portion. The socket is constructed to encapsulate more than half of the ball portion, thereby securing the ball portion in a movable relationship with respect to the socket.

Traditionally, the parts of these joints have been made from the same material. For example, in the field of medical implants, the most commonly utilized material has been cobalt-chromium alloys. These materials are advantageous for these uses because they are strong enough to withstand the day to day forces applied to then and they are light enough to be suitable as a replacement for the natural joint, among other suitable characteristics. However, the wear between the two parts has made the use of these devices, for long term applications, somewhat undesirable. One proposed solution has been to use different materials to construct the joint parts, wherein one material is tougher than the other material. This makes the replacement of a single part necessary instead of the replacement of both parts. However, an invasive surgery is still necessary to remove and replace the worn part and therefore, this solution still provides a substantial risk of injury to a patient- Furthermore, debris worn off of the softer part may be difficult to remove from the patient.

US-A-5,660,482 shows a sliding contact bearing, especially an aircraft landing gear trunnion beating, comprises two bearing members composed of titanium or titanium alloy with cooperating sliding surfaces a first one of which is chromium plated and the second one of which is coated with nickel and fluorocarbon polymer.

US-A-1,864,013 teaches an electrolytic deposition process where chromium in its hexavalent state is used in the deposition.

WO 01/05337 A1 shows a hip joint prostheses including an acetabular cup mounted in the hip socket of the pelvis. The prosthesis also includes a head which has a radius of curvature complementary to the cavity in the acetabular cup. The head is typically made of metal. A neck is connected to the head joining the head to the stern. The head and the acetabular cup are designed to allow a great deal of angular articulation. The bearing portions can be made with radiation treated ultrahigh molecular weight polyethylene polymer having substantially no detectable free radicals.

It is an object of the invention to provide a movable joint with a longer life time, and a method of making such a movable joint.

To achieve this object, the movable joint of the invention is constructed as claimed in the main method claim. Advantageous embodiments of the invention are characterized in the sub-claims.

The present invention offers a solution to this problem by providing a portion of the joint constructed having a chromium interface surface that reduces wear between the joint surfaces, such as both the ball and socket portions of a ball joint, by virtue of its intrinsic hardness and lubricity. The present invention generally provides a ball joint, having a ball portion comprising at least a deposition of chromium forming an outer surface of the ball portion. The ball portion is adapted to be rotatably captured within a defined area of the socket portion, thereby capturing the ball portion in the socket portion. In each embodiment, the chromium deposition forms an interface surface between the first and second portions.

According to the invention the chromium material utilized for deposition on either the first or second portion of a movable joint is comprised of hexavalent chromium. The chromium material may be in the form of an electro-chemical bound, thin deposit of chromium on the outer surface of the portion. Furthermore, the chromium may be bonded to the outer surface of the portion by electro-deposition.

In a ball-type joint, the socket portion generally has an area constructed and arranged to receive the ball portion in a movable relationship within the confines of the defined area. According to the invention, the socket portion of the joint is formed from ultra high molecular weight polyethylene. This material-provides a suitable and complimentary surface to that of a chromium deposited ball portion, thereby providing increased wear resistance to the device.

One application that joints, constructed according to the present invention, are particularly suited for is use in replacement of natural human or animal joints, such as knee, ankle, elbow, shoulder, spine, etc. However, the devices may be useful in any medical or non-medical application that, among other criteria, requires a joint with good wear resistance. Joints fabricated according to the present invention are also suited for these applications because they provide a reduction in fretting. Fretting is the production of wear debris through the interaction between two or more parts. The reduction of fretting reduces the chance of osteolysis, which occurs when wear debris enters the bloodstream.

The aforementioned benefits and other benefits including specific features of the invention will become clear from the following description by reference to the accompanying drawings in which:
- FIG. 1: is a cut-away side view of a prosthetic joint produced according to the present invention wherein the socket has been attached to the bone surface of a patient;
- FIG. 2: is a magnified cut-away side view of a portion of the ball of the implant of the embodiment of FIG. 1 showing the interface of chromium applied to the surface of the ball portion;
- FIG. 3: is a cut-away side view of an example not falling under the invention showing an interface of chromium applied to the surface of the socket portion;
- FIG. 4: is a cut-away side view of the embodiment of FIG. 3; and
- FIG. 5: is a cut-away side view of a prosthetic joint produced according to the present invention in assembled condition showing the interface of chromium applied to the surface the ball portion.

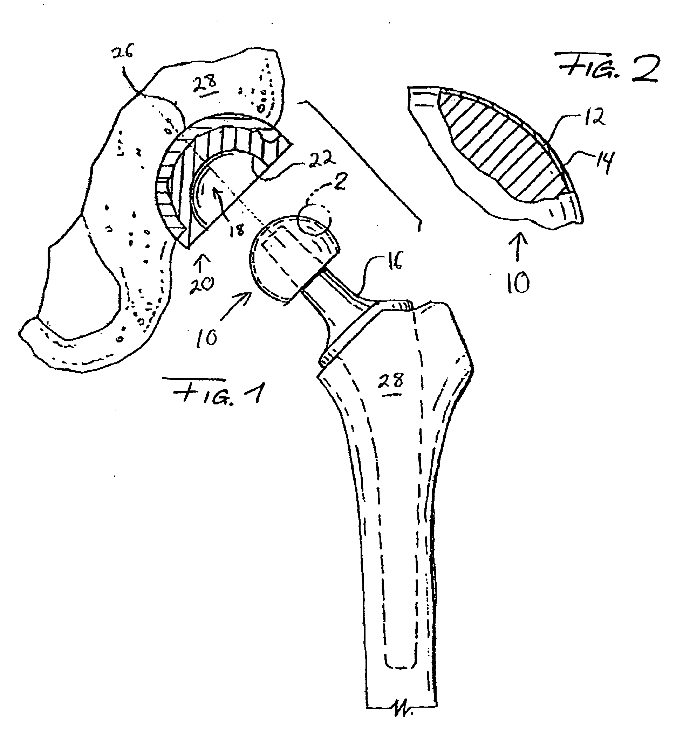

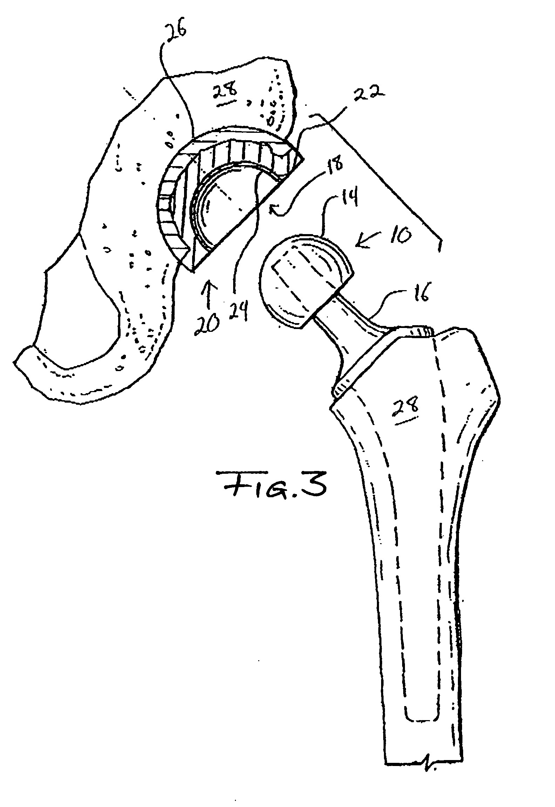

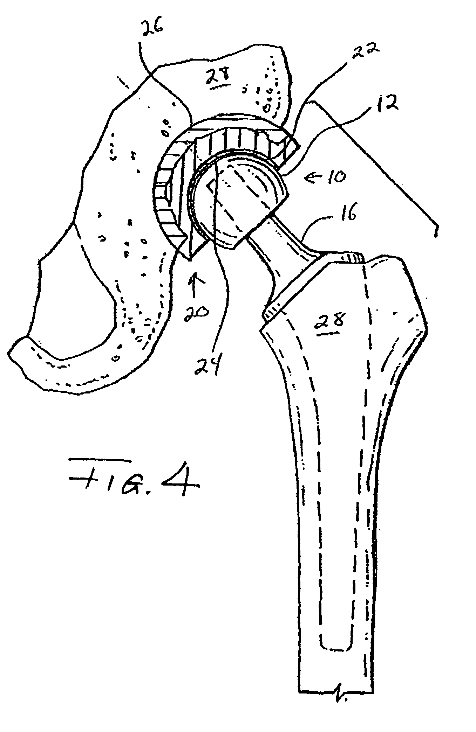

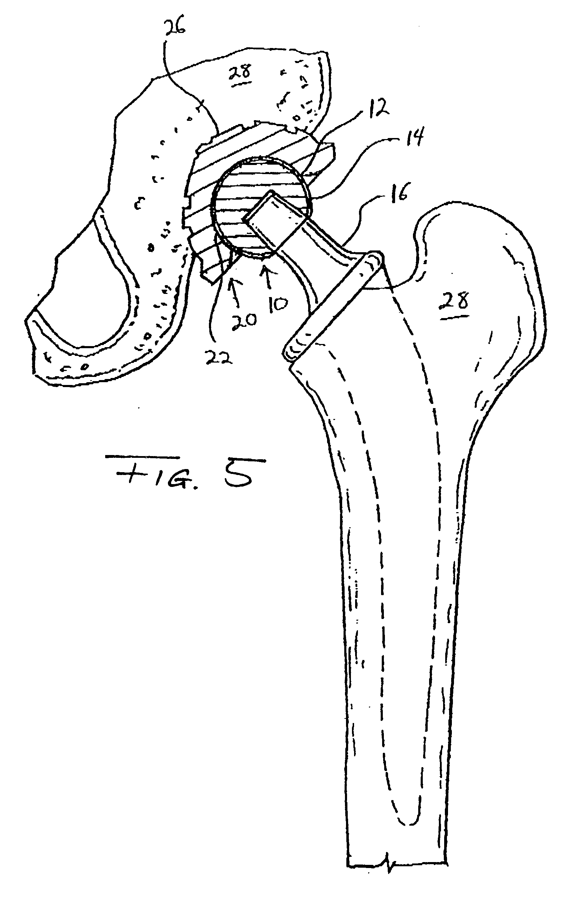

Referring now to the drawings wherein like reference numerals denote like elements throughout the several views, FIG. 1 illustrates a cut-away side view of an embodiment of the present invention. An embodiment of the present invention comprises a ball joint having a first, ball shaped, portion 10 having an outer surface 12 and a second, socket shaped, portion 20 having an outer surface 22. The ball portion 10 is sized and shaped to engage the cup 18 formed in the socket portion 20. As shown in Figs. 4 and 5, the cup 18 is an area constructed and arranged to hold the ball portion 10 within the confines of the cup 18 and to allow the ball portion 10 to rotate within the confines of the cup 18. The ball portion 10 is typically attached to a stem 16 that is enabled to move relative to the socket portion 20 because of the rotatable engagement of the ball portion 10 with the socket portion 20.

The socket portion 20 and stem 16 of the ball portion 10 may be attached to an attachment surface 28 by any means known in the art. Some suitable examples of attachment means include: mechanical attachment assemblies, such as screws and nuts and bolts, and adhesive mechanisms, such as cement and glue, for example.

Furthermore, the shape of the surface 26 of the socket portion 20 utilized for attachment to the attachment surface 28 may be of any suitable shape known in the art. For example, Figs. 1, 3, and 4 illustrate a socket surface 26 10 having a substantially uniform circular surface, whereas Fig. 5 illustrates a socket portion 20 having a non-uniform surface 26.

The surface coated with chromium material may be either the outer surface 12 of the first portion 10 or the outer surface 22 of the second portion 20. In the embodiment shown in Figs. 1, 2, and 5 a thin deposition of chromium is placed over the outer surface 12 of the first portion 10. In the comparative example not falling under the invention, shown in Figs. 3 and 4, a thin deposition of chromium is placed over the outer surface 22, generally formed within the cup 18, of the second portion 20.

By applying the chromium to one of the outer surfaces 12 (or 22), the chromium provides an interface between the materials used to form the first and second portions. The interface may be utilized with any materials that form the first and second portions known in the art. For example, cobalt-chromium alloys or stainless steel are two examples of materials that may be coated with chromium.

Additionally, when one of the first or second portions is coated with chromium, the other first or second portion may preferably be constructed from an ultra high molecular weight polyethylene material. For example, and according to the invention; a ball portion may be comprised of a cobalt-chromium alloy coated with a deposition of chromium and a socket portion may be constructed from ultra high molecular weight polyethylene. In a comparative example not falling under the invention, both the first and second portions may be formed of a cobalt-chromium based alloy with one of the surfaces of the two portions having a chromium deposition thereon.

The chromium utilized for the deposition process is hexavalent chromium and the deposition is preferably electro-chemically bound. The chromium may be deposited through any process known in the art, such as electro-deposition. The deposition may occur by flash coating the surface, thereby depositing the chromium thereon. One suitable thickness for the chromium deposition is approximately 5 µm (2/10,000 of an inch), however, the deposition may be as small as 1,3 µm (50/millionths of an inch). The process of applying the coating may also include pre and post plating mechanical polishing.

Since many possible embodiments may be made of the present invention without departing from the scope thereof, it is to be understood that all matter herein set forth or shown in the accompanying drawings is to be interpreted in the illustrative and not limiting sense.

## Claims

1. A method of producing a coated movable prosthetic joint, the steps of the method comprising:
providing a ball shaped first portion (10) being constructed from cobalt-chromium based alloy;
providing on said first portion (10) a deposition of chromium material utilizing hexavalent chromium in order to reduce fretting and forming an outer surface (12); and
providing a socket shaped second portion (20) from ultra high molecular weight polyethylene, said second portion (20) being adapted to interface with said outer surface (12) of said first portion (10) of said joint, thereby forming an interface between said first portion (10) and said second portion (20) such that said first and second portions (10, 20) are capable of rotatable movement with respect to each other.

2. The method according to claim 1, wherein said second portion (20) is constructed and arranged to receive said first portion (10) and has an outer surface (22).

3. The method according to claim 1, wherein said chromium deposition is provided in the form of an electro-chemically bound, thin deposit of chromium on said outer surface (12) of said first portion (10).

4. The method according to claim 1, wherein said chromium is bonded to said outer surface by electro-deposition.

## Patentansprüche

1. Verfahren zur Herstellung eines beschichteten, bewegbaren, prothetischen Gelenks, wobei die Schritte des Verfahrens umfassen:
- Bereitstellen eines kugelförmigen, ersten Abschnitts (10), der aus einer Legierung auf der Basis Kobaltchrom hergestellt ist;
- Bereitstellen einer Schicht aus Chrommaterial unter Verwendung von hexavalentem Chrom auf dem ersten Abschnitt (10), um eine Reibkorrosion zu reduzieren und eine Außenfläche (12) zu bilden; und
- Bereitstellen eines sockelförmigen, zweiten Abschnitts (20) aus Polyethylen mit ultrahohem Molekulargewicht, wobei der zweite Abschnitt (20) in der Lage ist, mit der Außenfläche (12) des ersten Abschnitts (10) des Gelenks einen Kontakt zu bilden, wodurch eine Kontaktzone zwischen dem ersten Abschnitt (10) und dem zweiten Abschnitt (20) gebildet wird, so dass der erste und der zweite Abschnitt (10, 20) eine Rotationsbewegung in Bezug aufeinander ausführen können.

2. Verfahren nach Anspruch 1, worin der zweite Abschnitt (20) so hergestellt und angeordnet ist, dass er den ersten Abschnitt (10) aufnimmt, und dass er eine Außenfläche (22) hat.

3. Verfahren nach Anspruch 1, worin die Chromschicht in der Form einer elektrochemisch gebundenen, dünnen Chromschicht auf der Außenfläche (12) des ersten Abschnitts (10) vorgesehen ist.

4. Verfahren nach Anspruch 1, worin das Chrom durch Elektroabschaltung mit der äußeren Oberfläche verbunden ist.

## Revendications

1. Procédé pour produire une articulation prosthétique recouverte, les étapes du procédé comprenant:
- pourvoir une première portion sphérique (10) étant construite d'un alliage basé sur cobalt-chrome;
- pourvoir sur ladite première portion (10) d'une déposition de chrome utilisant le chrome hexavalent pour réduire l'usure par frottement et formant une surface extérieure (12); et
- pourvoir une seconde portion en forme de logis (20) de polyéthylène de poids moléculaire ultra-haut, ladite seconde portion (20) étant adaptée pour faire interface avec ladite surface extérieure (12) de ladite première portion (10) de ladite articulation, de cette manière formant une interface entre ladite première portion (10) et ladite seconde portion (20) de sorte que lesdites première et seconde portions (10, 20) soient capables de mouvement rotatif l'une à l'égard de l'autre.

2. Procédé selon la revendication 1, où ladite seconde portion (20) est construite et arrangée pour recevoir ladite première portion (10) et présente une surface extérieure (22).

3. Procédé selon la revendication 1, où ladite déposition de chrome est pourvue dans la forme d'un dépôt mince de chrome, lié par procédé électrochimique sur ladite surface extérieure (12) de ladite première portion (10).

4. Procédé selon la revendication 1, où ledit chrome est lié à ladite surface extérieure par électrodéposition.
